(19) Europäisches Patentamt
European Patent Office
Office européen des brevets

(11) EP 3 107 892 B1

# (12) EUROPÄISCHE PATENTSCHRIFT

(45) Veröffentlichungstag und Bekanntmachung des Hinweises auf die Patenterteilung:
**08.11.2017 Patentblatt 2017/45**

(21) Anmeldenummer: **15704754.9**

(22) Anmeldetag: **06.02.2015**

(51) Int Cl.:
***C07C 209/26*** *(2006.01)* ***C07C 211/08*** *(2006.01)*

(86) Internationale Anmeldenummer:
**PCT/EP2015/052500**

(87) Internationale Veröffentlichungsnummer:
**WO 2015/124442 (27.08.2015 Gazette 2015/34)**

## (54) VERFAHREN ZUR HERSTELLUNG VON N-ETHYL-DIISOPROPYLAMIN

METHOD FOR THE PRODUCTION OF N-ETHYL-DIISOPROPYLAMINE

PROCÉDÉ DE FABRICATION DE N-ÉTHYL-DIISOPROPYLAMINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.02.2014 EP 14155557**

(43) Veröffentlichungstag der Anmeldung:
**28.12.2016 Patentblatt 2016/52**

(73) Patentinhaber: **BASF SE**
**67056 Ludwigshafen am Rhein (DE)**

(72) Erfinder: **DE WINNE, Hendrik**
**B-2800 Mechelen (BE)**

(74) Vertreter: **BASF IP Association**
**BASF SE**
**G-FLP-C006**
**67056 Ludwigshafen (DE)**

(56) Entgegenhaltungen:
**EP-A1- 1 020 424**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 107 892 B1

## Beschreibung

Die vorliegende Erfindung betrifft ein Verfahren zur Herstellung von N-Ethyl-diisopropylamin (EDIIPA, Hünig-Base) der Formel

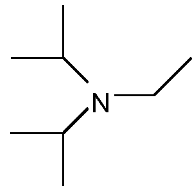

durch Umsetzung von Acetaldehyd mit Diisopropylamin (DIIPA) und Wasserstoff bei erhöhter Temperatur und unter Druck in Gegenwart eines Hydrierkatalysators.

N-Ethyl-diisopropylamin ist ein wichtiges Amin, das als starke, wenig nukleophile Base in Eliminierungsreaktionen eingesetzt wird und als Katalysator bzw. Hilfsbase in der organischen Synthese von beispielsweise Wirkstoffen (siehe z.B. WO 98/07430 A) Verwendung findet.

In Chem. Ber. 91, Seiten 380-392 (1958), wird die Synthese von N-Ethyl-diisopropylamin durch Umsetzung von Diisopropylamin mit Diethylsulfat beschrieben. Hierbei fallen Sulfate als unerwünschte Nebenprodukte an, die anschließend aufwendig entsorgt werden müssen.

Auch die in J. Org. Chem. 16, Seiten 1911-1920, (1951) und US 2,692,285 A beschriebene Umsetzung des Diisopropylamins mit Ethyliodid zur Synthese von N-Ethyl-diisopropylamin führt zu unerwünschten Salzen als Nebenprodukten.

JP 10081650 A2, erteilt als JP 2851274 B2 (Koei Chem.), sowie JP 02180854 A2, erteilt als JP 2740828 B2 (Koei Chem.), beschreiben insbesondere die Herstellung von N-Ethyl-diisopropylamin aus Acetaldehyd und Diisopropylamin oder aus Aceton und Ethylamin an Pd/C-Suspensionskatalysatoren in semibatch-Fahrweise bei 20 - 200 °C und bevorzugt 5 - 60 atm.

EP 1 020 424 A1 (BASF AG) beschreibt ein Verfahren zur Herstellung von N-Ethyl-diisopropylamin durch Umsetzung von Acetaldehyd mit Diisopropylamin und Wasserstoff bei erhöhter Temperatur und unter Druck in Gegenwart eines Hydrierkatalysators, das dadurch gekennzeichnet, dass der Katalysator ein oxidisches Trägermaterial, ausgewählt aus der Gruppe Zirkoniumdioxid, Titandioxid, Aluminiumoxid, Siliziumdioxid, Zinkoxid, Magnesiumoxid, Cerdioxid, Tone und Zeolithe oder Mischungen daraus, enthält. Bevorzugte Katalysatoren sind z.B. $Pd/Al_2O_3$, $Pt/ZrO_2$, $Pd+Ag/SiO_2$, $Ru/Al_2O_3$, $Pd/ZrO_2$.

WO 2007/137990 A1 (BASF AG) lehrt Verfahren zur Herstellung eines Amins durch Umsetzung eines Aldehyds und/oder Ketons mit Wasserstoff und einer Stickstoffverbindung, ausgewählt aus der Gruppe primärer und sekundärer Amine, in Gegenwart eines Heterogenkatalysators, wobei die Umsetzung unter Einsatz eines Suspensionskatalysators als Heterogenkatalysator erfolgt und in semibatch-Fahrweise durchgeführt wird, bei der die Stickstoffverbindung als ein Reaktionspartner im Reaktionsgefäß vorgelegt und der Aldehyd und/oder das Keton als der andere Reaktionspartner im Reaktionsverlauf zugegeben wird, und im Reaktionsverlauf in Abhängigkeit vom erreichten Umsatz der Stickstoffverbindung der Aldehyd und/oder das Keton portionsweise oder kontinuierlich zum Reaktionsgemisch gegeben wird, bis ein Umsatz der Stickstoffverbindung von mindestens 95 % resultiert, und der Katalysator ganz oder teilweise nach der Reaktionspartie für die nächste Reaktionspartie zum erneuten Einsatz im Reaktionsgefäß verbleibt und wieder eingesetzt wird.

Eine besondere Ausführungsform ist u.a. die Herstellung von Hünig-Base durch Umsetzung mit Acetaldehyd mit Diisopropylamin unter Einsatz eines Pd/C-Suspensionskatalysators (loc. cit., Seite 14 unten).

Der vorliegenden Erfindung lag die Aufgabe zugrunde, unter Überwindung eines Nachteils oder mehrerer Nachteile des Stands der Technik, ein verbessertes wirtschaftliches, vor allem diskontinuierlich ausführbares Verfahren zur Herstellung von N-Ethyl-diisopropylamin mit guter Selektivität, Ausbeute und Raum-Zeit-Ausbeute (RZA) aufzufinden. Es sollte als Edukt ein Amin als Rohware eingesetzt werden können, d.h. eine Aminware, die nicht zuvor aufwändig reindestilliert werden muss.

Demgemäß wurde ein Verfahren zur Herstellung von N-Ethyl-diisopropylamin durch Umsetzung von Acetaldehyd mit Diisopropylamin und Wasserstoff bei erhöhter Temperatur und unter Druck in Gegenwart eines Heterogen-Hydrierkatalysators, wobei es sich bei dem Katalysator um einen geträgerten Übergangsmetallkatalysator handelt, der als katalytisch aktives Metall Pd und/oder Pt enthält, gefunden, welches dadurch gekennzeichnet ist, dass das eingesetzte Diisopropylamin eine Reinheit von 58 bis 94 Gew.-% und Verunreinigungen wie folgt aufweist: 3 bis 20 Gew.-% Wasser, 3 bis 20 Gew.-% Isopropanol, 0 bis 2 Gew.-% Sonstige.

Setzt man als Edukt eine verunreinigte Rohware ein, so ist es in der Regel der Fall, dass auch das Produkt

der Umsetzung vermehrt Komponenten als Verunreinigung enthält. Eine oder mehrere dieser Verunreinigungen lassen sich dann oft nur aufwändig, mit hohen Ausbeuteverlust oder gar nicht aus dem Produkt abtrennen. Eine hohe Produktreinheit ist jedoch oft erforderlich; so findet das N-Ethyl-diisopropylamin Anwendung in der Synthese von pharmazeutischen Wirkstoffen.

Erfindungsgemäß wurde überraschenderweise gefunden, dass man rohes Diisopropylamin mit einem Gehalt an Wasser und an Isopropanol einsetzen kann, welches in der Umsetzung mit Acetaldehyd nicht zu bedeutend mehr Ethanolbildung und Ausbeuteverlust, verglichen mit dem Einsatz von reinerem Diisopropylamin, führt. So würde man nach dem Prinzip von Le Chatelier erwarten, dass die Anwesenheit von Wasser in der Reaktionsmischung zu einer Verschiebung des Reaktionsgleichgewichts zuungunsten des Enamins, dessen Hydrierung in der Folge das Zielprodukt ergibt, führt (vgl. folgendes Reaktionsschema), wodurch relativ gesehen mehr Acetaldehyd direkt zu Ethanol hydriert würde.

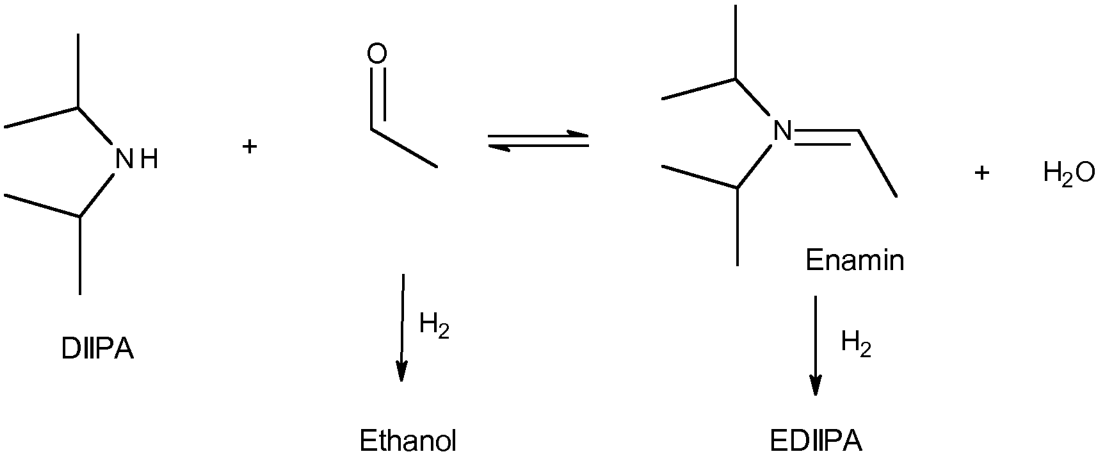


Gemäß vorliegender Erfindung sind die Bedingungen und Katalysator so gewählt, dass es nur in sehr geringem Ausmaß oder besonders gar nicht zu dieser höheren Ethanolbildung kommt. Außerdem gibt es überraschenderweise unter den erfindungsgemäßen Bedingungen keine bedeutende Acetalbildung durch die Reaktion von Isopropanol mit Acetaldehyd (vgl. folgendes Reaktionsschema). Weiterer Ausbeuteverlust durch diese Acetalbildung tritt also vorteilhafterweise ebenfalls nur in sehr geringem Ausmaß oder besonders gar nicht auf.

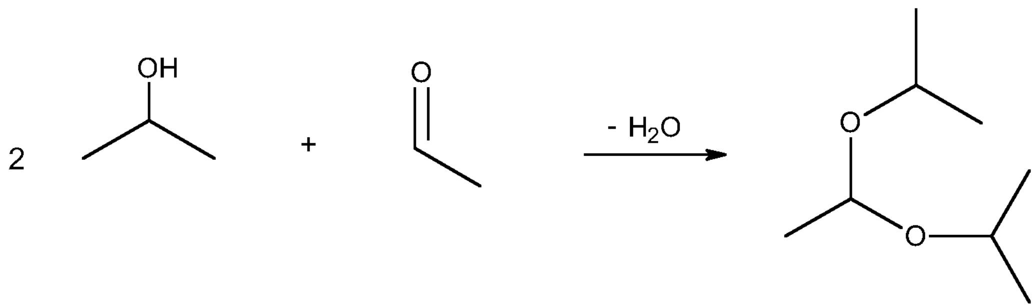


Acetal

Weiterhin sind die entstehenden, von Isopropanol abgeleiteten Nebenkomponenten (z.B. N,N-Diisopropyl-n-butylamin, N,N-Diisopropyl-(4-amino-1-butanol)) sehr gut abtrennbar, insb. sehr gut destillativ abtrennbar. Mittels des erfindungsgemäßen Verfahrens kann man, obwohl ein rohes Diisopropylamin eingesetzt wird, trotzdem mit hoher Ausbeute ein sehr reines Endprodukt (N-Ethyl-diisopropylamin) erhalten, das geeignet ist als Einsatzstoff z.B. für die Synthese von pharmazeutischen Wirkstoffen.

Das im erfindungsgemäßen Verfahren eingesetzte Diisopropylamin weist eine Reinheit von nur 58 bis 94 Gew.-% und Verunreinigungen wie folgt auf:

3 bis 20 Gew.-% Wasser, 3 bis 20 Gew.-% Isopropanol, 0 bis 2 Gew.-% Sonstige.

Bevorzugt weist das im erfindungsgemäßen Verfahren das eingesetzte Diisopropylamin eine Reinheit von nur

62,5 bis 90 Gew.-% und Verunreinigungen wie folgt auf:

5 bis 18 Gew.-% Wasser, 5 bis 18 Gew.-% Isopropanol, 0 bis 1,5 Gew.-% Sonstige.

Weiter bevorzugt weist das im erfindungsgemäßen Verfahren das eingesetzte Diisopropylamin eine Reinheit von nur 69,0 bis 85,9 Gew.-% und Verunreinigungen wie folgt auf:

7 bis 15 Gew.-% Wasser, 7 bis 15 Gew.-% Isopropanol, 0,1 bis 1,0 Gew.-% Sonstige.

(Alle Gew.-%-Angaben bezogen auf das Gewicht des eingesetzten rohen Diisopropylamins).

Bei den Sonstigen handelt es sich insbesondere um N- und/oder O-haltige organische Verbindungen, wie besonders in den unten genannten Ausführungsbeispielen aufgeführt. Das eingesetzte rohe Diisopropylamin wurde zuvor als Nebenprodukt einer Umsetzung von Isopropanol mit Ammoniak zu Monoisopropylamin (Alkoholaminierung) gewonnen, wobei in einer weiteren bevorzugten Ausgestaltung das dort eingesetzte Isopropanol zuvor durch eine Umsetzung von Aceton mit Wasserstoff (Hydrierung) erhalten wurde.

Solche Umsetzungen sind in WO 2013/075974 A (BASF SE) beschrieben.

Bevorzugt ist die Umsetzung von Isopropanol mit Ammoniak zu Monoisopropylamin (MIPA) in Gegenwart von $H_2$ bei 20 bis 80 bar, 150 bis 225 °C, einem 1,5- bis 5-fachen molaren Überschuss von $NH_3$ bezogen auf Isopropanol und einem Cu-haltigen Heterogenkatalysator, insb. dem in EP 696 572 A (BASF AG) offenbarten Katalysator, dessen katalytisch aktive Masse vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$, enthält, beispielsweise dem in loc. cit., Seite 8, offenbarten Katalysator mit der Zusammensetzung 31,5 Gew.-% $ZrO_2$, 50 Gew.-% NiO, 17 Gew.-% CuO und 1,5 Gew.-% $MoO_3$. Die Katalysatorbelastung liegt z.B. im Bereich von 0,10 bis 0,14 kg Isopropanol / ($l_{Kat.}$ • h), ($l_{Kat.}$ = Schüttvolumen).

Die erhaltene Reaktionsmischung als Produkt der Umsetzung von Isopropanol mit Ammoniak zu Monoisopropylamin wird bevorzugt destillativ, besonders wie folgt destillativ aufgearbeitet durch

1. Abtrennung von Ammoniak ($NH_3$),
2. Abtrennung von MIPA,
3. Abtrennung von höher als MIPA und DIIPA siedenden Nebenkomponenten,
4. Abtrennung von ternärem Azeotrop Isopropanol-DIIPA-Wasser.

Bevorzugt ist die Umsetzung von Aceton mit Wasserstoff zu Isopropanol bei 30 bis 90 bar, 50 bis 160 °C, einem 1- bis 3-fachen molaren Überschuss von $H_2$ bezogen auf Aceton und in Gegenwart eines ein Cu-haltigen Heterogenkatalysators, insb. einem Aluminiumoxid-geträgerten Cu-Katalysator, z.B. dem in EP 563 327 A = WO 92/10290 A1 (Engelhard Corp.) beschriebenen geformten Kupferchromit(III)-Katalysator, hergestellt aus einer Mischung, umfassend 20 bis 80 Gew.-% Kupferchromit(III), worin bevorzugt ein Teil oder das gesamte Kupferchromit(III) die Formel CuO • CuCr204 hat, und 20 bis 80 Gew.-% von mindestens einem extrudierbaren anorganischen Bindemittelmaterial, worin der Katalysator eine Oberfläche von 20 bis 225 $m^2/g$ hat und das Gesamt-Porenvolumen der Poren mit einem Durchmesser bis zu 9500 Nanometer (95000 Angström) in dem Katalysator 0,35 bis 1 $cm^3/g$ beträgt.

Der im erfindungsgemäßen Verfahren eingesetzte geträgerte Übergangsmetallkatalysator wird bevorzugt als Suspensionskatalysator eingesetzt, der Heterogen-Katalysator befindet sich dann also suspendiert in einer flüssigen Phase des Reaktionsgemisches.

Erfindungsgemäß bevorzugte Übergangsmetallkatalysatoren sind solche, die als Aktivkomponenten eines oder mehrere Metalle ausgewählt aus der Gruppe der Metalle Pd und Pt enthalten. Besonders bevorzugt ist das Metall Pd als zumindest eine, insbesondere einzige, Aktivkomponente. (Aktivkomponente = katalytisch aktive Komponente).

Trägermaterialien der erfindungsgemäß einzusetzenden Katalysatoren sind bevorzugt Aktivkohle oder Aluminiumoxid, besonders bevorzugt Aktivkohle.

Ein im Rahmen der vorliegenden Erfindung besonders bevorzugter Katalysator ist Pd auf Aktivkohle (Pd/C).

Die genannten Katalysatoren weisen vorteilhafterweise einen Gehalt an Pd und/oder Pt, von 0,1 bis 25 Gew.-%, bevorzugt von 0,5 bis 15 Gew.-% und besonders bevorzugt von 4 bis 11 Gew.-% [jeweils bezogen auf das reduzierte Metall bzw. die reduzierten Metalle (Oxidationsstufe 0) des fertigen Katalysators und bezogen auf das Gesamtgewicht des trockenen Katalysators] auf.

Derartige Katalysatoren sind kommerziell zugänglich und beispielsweise unter den Bezeichnungen Degussa E1002, Degussa E101, Degussa E105, Degussa E106, Engelhard C3630, Heraeus K201, Heraeus K202, Heraeus K203, Heraeus K204, Heraeus K219 erhältlich.

Der gewählte Katalysator wird vorteilhaft in einer solchen Menge eingesetzt, dass die Menge an Katalysator (wasserfrei gerechnet) bezogen auf die Menge an eingesetztem Diisopropylamin im Bereich von 0,1 bis 20,0 Gew.-%, besonders im Bereich von 0,5 bis 5,0 Gew.-%, beträgt.

Der Suspensionskatalysator kann besonders einen Wassergehalt im Bereich von 1 bis 150 Gew.-%, weiter besonders im Bereich von 3 bis 10 Gew.-%, (jeweils bezogen auf das Gewicht des trockenen Katalysators) aufweisen.

In einer bevorzugten Ausführungsform wird die Umsetzung im erfindungsgemäßen Verfahren ohne Zusatz von Promotoren im Katalysator, wie z.B. Zink-Dotierungen, oder Hilfsstoffen, wie z.B. Kohlenmonoxid, durchführt.

Gemäß einer Ausführungsform der Erfindung wird die Umsetzung in der Flüssigphase oder in einer gemischten Flüssig-/Gasphase mit mindestens 50 Gew.-% des Reaktionsgemisches in der Flüssigphase durchgeführt.

Als Reaktoren können z.B. Rührkessel, Autoklaven, Schlaufenreaktoren oder gepackte Blasensäulen verwendet werden. Bevorzugter Reaktor ist ein Rührkessel.

Das erfindungsgemäße Verfahren lässt sich kontinuierlich oder besonders bevorzugt diskontinuierlich durchführen, wobei der Katalysator bevorzugt als Suspensionskatalysator im Reaktor angeordnet ist.

Das Verfahren kann in der Flüssigphase oder in der Gasphase durchgeführt werden. Bevorzugt ist eine zumindest teilweise vorhandene, besonders weit überwiegende, Flüssigphase. Je nach den gewählten Reaktionsbedingungen (Druck, Temperatur) wird ein gewisser Anteil der Edukte entsprechend dem Partialdruck gasförmig vorliegen.

Die beiden Edukte Diisopropylamin und Acetaldehyd können in einem stöchiometrischen, über- oder unterstöchiometrischen Molverhältnis eingesetzt werden. Bevorzugt wird die Umsetzung in Gegenwart von überschüssigem Acetaldehyd durchgeführt.

Das Molverhältnis Acetaldehyd zu Diisopropylamin (berechnet 100 %) liegt bevorzugt bei 1,0 bis 5,0 mol/mol, besonders bei 1,1 bis 2,0 mol/mol, weiter besonders bei 1,2 bis 1,5 mol/mol.

Besonders bevorzugt ist eine Semibatch-Fahrweise, bei der das Amin und der Katalysator vorgelegt werden und anschließend der Aldehyd bei Reaktionstemperatur und -druck zudosiert wird.

Die Zugabe erfolgt, bevorzugt innerhalb von 0,5 bis 24 Stunden, weiter bevorzugt innerhalb von 1 bis 15 Stunden, kontiniuierlich oder portionsweise. Zu aufeinanderfolgenden Zeitpunkten, z.B. alle 30 Min. oder alle 15 Min., oder kontinuierlich, z.B. per online-Gaschromatographie oder online-Spektroskopie, wird der Umsatz geprüft. Der Umsatz wird z.B. überprüft, indem eine kleine Probemenge aus dem Reaktor entnommen wird und die Zusammensetzung analysiert wird, beispielweise mittels Gaschromatographie. Der Umsatz (U) errechnet sich aus dem Quotienten der Mengenanteile der aus der eingesetzten Stickstoffverbindung gebildeten Produkte und der Summe der Mengenanteile der aus der Stickstoffverbindung gebildeten Produkte und nicht-umgesetzter Stickstoffverbindung gemäß folgender Formel:

$$U = \frac{n_2}{n_2 + n_1} * 100\%$$

mit $n_2$ = Stoffmenge der aus der Stickstoffverbindung gebildeten Produkte und
$n_1$ = Stoffmenge an nicht-umgesetzter Stickstoffverbindung

Der Aldehyd wird bevorzugt solange dosiert, bis ein Umsatz von mindestens 95 %, bevorzugt mindestens 96 %, besonders bevorzugt mindestens 97 %, z.B. 97,5 bis 99,8 %, jeweils bezüglich eingesetzter Stickstoffverbindung, erreicht ist.

In einer besonders bevorzugten Ausführungsform der Erfindung wird die Zugabegeschwindigkeit des Aldehyds so gewählt, dass die jeweils gewünschte Maximaltemperatur (bevorzugt im Bereich von 70 bis 180 °C) der Reaktion nicht überschritten wird. (Geschwindigkeit der Zugabe z.B. in Mol Aldehyd pro 30 Min.).

Bei Zugabe von Aldehyd zur Stickstoffverbindung erfolgen Reaktionen, in denen Wärme freigesetzt wird: Das Zwischenprodukt Enamin wird in einer exothermen Reaktion unter Wasserabspaltung gebildet. Das Zwischenprodukt wird dann in einer weiteren exothermen Reaktion in Anwesenheit von Wasserstoff und dem Hydrierkatalysator zum Produkt umgesetzt (Hydrierung der Doppelbindung). Die Reaktionstemperatur kann auf eine sehr einfache Weise kontrolliert und begrenzt werden, indem die Zugabe des Aldehyds vor Erreichen einer zuvor festgelegten Maximaltemperatur verlangsamt oder kurzzeitig unterbrochen wird, bis die Reaktionsmischung durch die Wärmeabstrahlung des Reaktionsgefäßes oder unter Anwendung einer externen Kühlung wieder erniedrigt wurde.

Nach Abschluss der Reaktion lässt man den Katalysator im Reaktionsgefäß absetzen, wobei sich zwei Phasen (eine organische und eine wässrige Phase) im Reaktionsgemisch ausbilden. Die organische Phase und bevorzugt ein Teil der wässrigen Phase, wird dann, bevorzugt über ein Filter, aus dem Reaktor gefahren.

Die Reaktionstemperatur kann sich nach der Aktivität des Katalysators richten. Die Reaktionstemperatur liegt bevorzugt im Bereich von 15 bis 180 °C, bevorzugt 30 bis 170 °C, besonders bevorzugt 70 bis 160 °C, weiter bevorzugt 100 bis 140 °C.

Das erfindungsgemäße Verfahren wird bevorzugt bei einem Absolutdruck (= Reaktionsdruck) im Bereich von

1 bis 120 bar, bevorzugt 5 bis 100 bar, besonders bevorzugt 10 bis 80 bar, durchgeführt. Der Druck im Reaktor, welcher sich aus der Summe der Partialdrücke des Amin-Edukts, des Acetaldehyds, der Verunreinigungen und der gebildeten Reaktionsprodukte bei den angegebenen Temperaturen ergibt, wird zweckmäßigerweise durch Aufpressen von Wasserstoff auf den gewünschten Reaktionsdruck erhöht.

Bevorzugt wird eine Abgasmenge von 0,1 bis 400 Normkubikmeter / (h • (Liter Reaktionsvolumen)), insbesondere 1 bis 20 Normkubikmeter / (h • (Liter Reaktionsvolumen)), gefahren. (Normkubikmeter = Volumen unter Normalbedingungen (1 bar abs., 20 °C)).

Die Anwendung höherer Temperaturen, höherer Gesamtdrücke und kleinerer Katalysatormengen ist möglich.

Die Aufarbeitung des Reaktionsaustrags und Isolierung des Verfahrensproduktes kann nach den üblichen Methoden, z. B. durch fraktionierte kontinuierliche oder diskontinuierliche Destillation bzw. Rektifikation erfolgen. Die Rektifikation kann beispielsweise bei Normaldruck (1 bar abs.) oder geringem Unter- oder Überdruck, z.B. bei Rücklaufverhältnissen von 1:1 bis 10:1 und z.B. in Kolonnen mit 5 bis 60 theoretischen Böden erfolgen.

Die Aufarbeitung des Reaktionsaustrags erfolgt bevorzugt wie folgt:

1) Abfiltrieren des Katalysators,
2) Phasentrennung von organischer und wässriger Phase,
3) Abtrennung von verbleibendem Wasser durch Destillation,
4) Abtrennung von leichter als N-Ethyl-diisopropylamin (EDIIPA) siedenden Nebenkomponenten durch Destillation,
5) Abtrennung von höher als EDIIPA siedenden Nebenkomponenten durch Destillation
6) Reindestillation von EDIIPA.

Die Destillationsausbeute liegt in der Regel bei > 80 %, besonders bei > 90 %.

Alle Druckangaben beziehen sich auf den Absolutdruck.

Beispiele und Vergleichsbeispiel

1) Umsetzung von Diisopropylamin (DIIPA) roh (Zwangsanfall aus einer Anlage zur Herstellung von MIPA) sowie Diisopropylamin (DIIPA) rein
jeweils mit Acetaldehyd an einem Pd/C-Katalysator (5 Gew.-% Pd auf Kohle) zu EDIIPA (Hünig-Base)

Das eingesetzte DIIPA roh entstand als Zwangsanfall bei der Aminierung von Isopropanol mit $NH_3$ zu Monoisopropylamin (MIPA) analog dem Verfahren wie es in WO 2013/075974 A (BASF SE) beschrieben ist. Dafür wurde Isopropanol (0,14 kg/l•h) umgesetzt mit $NH_3$ (Molverhältnis $NH_3$: iPrOH = 2,4) bei 46 bar und 196 °C. Als Katalysator kam der in der o.g. EP-Anmeldung zitierte und aus der EP 696 572 A (BASF AG) bekannte Katalysator, dessen katalytisch aktive Masse vor der Reduktion mit Wasserstoff die Zusammensetzung 31,5 Gew.-% $ZrO_2$, 50 Gew.-% NiO, 17 Gew.-% CuO und 1,5 Gew.-% $MoO_3$ aufweist, zum Einsatz. Die Reaktionsmischung wurde destillativ aufgearbeitet durch

1. Abtrennung von $NH_3$,
2. Abtrennung von MIPA,
3. Abtrennung von höher als MIPA und DIIPA siedenden Nebenkomponenten,
4. Abtrennung von ternärem Azeotrop Isopropanol-DIIPA-Wasser.

DIIPA bildet mit Wasser und Isopropanol ein ternäres Azeotrop. Eine vollständige Abtrennung von Isopropanol und Wasser wird deswegen destillativ ohne weitere Maßnahmen (Brechen des Azeotropes mit NaOH oder durch Pressure Swing Destillation) nicht erreicht.

In diesem Beispiel entstand dabei DIIPA roh mit einer DIIPA roh Konzentration von 80,0 Gew.-%.

Zusammensetzung des DIIPA roh (in Gew.-%):

| | |
|---|---|
| Wasser | 9,75 |
| Isopropanol | 10,05 |
| Diisopropylamin | 80,0 |
| Sonstige | 0,2 |

Das eingesetzte Isopropanol war zuvor durch Umsetzung von Aceton mit Wasserstoff bei 30 bis 90 bar, 50 bis 160 °C, einem 1- bis 3-fachen molaren Überschuss von $H_2$ bezogen auf Aceton, an einem Aluminiumoxid-geträgerten

Cu-Katalysator (29 Gew.-% CuO, 31 Gew.-% $Cr_2O_3$, 9 Gew.-% BaO, 30 Gew.-% $Al_2O_3$), wie beschrieben in EP 563 327 A = WO 92/10290 A1 (Engelhard Corp.), erhalten worden.

Die Reaktion mit Acetaldehyd wurde in semibatch-Fahrweise durchgeführt. DIIPA (wie oben erhalten) bzw. DIIPA rein (Vergleich) und der Pd/C-Katalysator (6 Gew.-% feucht, bez. auf DIIPA ber. 100 %) wurden jeweils bei 120 °C und 25 bar Wasserstoff in dem Reaktionsbehälter (0,3 l Autoklav) vorgelegt und Acetaldehyd (1,2 Mol-Äquivalente bez. auf DIIPA ber. 100 %ig) wurden innerhalb 3 h zudosiert. Anschließend wurde 3 h bei Reaktionstemperatur und -druck nachgerührt. Nach Abkühlen und entspannen wurden die Phasen getrennt und einzeln mittels GC analysiert. Nach Wasserbestimmung mittels Karl-Fischer-Titration wurden die Zusammensetzungen der organischen Phasen auf 100 Gew.-% normiert. Es wurde jeweils eine Syntheseausbeute von ca. 80 %, bezogen auf eingesetztes DIIPA ber. 100 %ig, erreicht. Die Zusammensetzung des erhaltenen roh EDIIPA ex DIIPA rein und roh EDIIPA ex DIIPA roh war wie folgt (in Gew.-%):

| Komponente | roh EDIIPA ex DIIPA roh | roh EDIIPA ex DIIPA rein |
|---|---|---|
| Ethanol | 0,410 | 0,232 |
| Isopropanol | 5,640 | 0,063 |
| n-Propylamin | 0 | 0,011 |
| n-Butanal | 0,480 | 0,344 |
| n-Butanol | 0,357 | 0,243 |
| DIIPA | 1,274 | 0,205 |
| Enamin | 0 | 0,020 |
| Isopropyl-n-propylamin | 0,004 | 0 |
| 2-Ethylbutanal | 0,362 | 0,288 |
| DEIPA | 0,194 | 0,434 |
| EDIIPA | 82,508 | 93,133 |
| EIPPA | 0,088 | 0,028 |
| DMeBBA | 0,101 | 0,000 |
| DIIP-BA | 6,049 | 4,063 |
| DIIPABol | 1,210 | 0,123 |
| N-Hexyl-DIIPA | 0,343 | 0,192 |
| N-Nonyl-DIIPA | 0,023 | 0,011 |
| Weitere Sonstige | 0,831 | 0,580 |

DEIPA = N,N-Diethyl-isopropylamin
EIPPA = N-Ethyl-N-isopropyl-n-propylamin
DMeBBA = N-(1,3-Dimethyl-butyl)-n-butylamin
DIIP-BA = N,N-Diisopropyl-n-butylamin
DIIPABol = N,N-Diisopropyl-(4-amino-1-butanol)

Das im Vergleichsversuch eingesetzte DIIPA rein hatte folgende Zusammensetzung (in Gew.-%):

| | |
|---|---|
| Wasser | 0,01 |
| Isopropanol | 0,00 |
| Diisopropylamin | 99,88 |
| Sonstige | 0,11 |

Aus den Beispielen ist zu sehen, dass nicht bedeutend mehr Ethanol entsteht, wenn schon bei Anfang der Reaktion Wasser anwesend ist, und dass praktisch kein Acetal (mögliche Reaktion von Isopropanol mit Acetaldehyd) und Folgeprodukte auftreten.

Die Aufarbeitung des Reaktionsaustrags erfolgte jeweils wie folgt:

1) Abfiltrieren des Katalysators,
2) Phasentrennung von organischer und wässriger Phase,
3) Abtrennung von verbleibendem Wasser durch Destillation,
4) Abtrennung von leichter als N-Ethyl-diisopropylamin (EDIIPA) siedenden Nebenkomponenten durch Destillation,
5) Abtrennung von höher als EDIIPA siedenden Nebenkomponenten durch Destillation,

6) Reindestillation von EDIIPA.

Alle neue Nebenkomponenten, inklusive die von Isopropanol abgeleiteten Komponenten, sind destillativ leicht abtrennbar. Dadurch war es, trotz eingesetztem DIIPA roh, bei der Aufarbeitung möglich, eine Destillationsausbeute von oberhalb 80 % zu erreichen.

Die gaschromatographisch ermittelte Zusammensetzung des erhaltenen reinen EDIIPA ex DIIPA rein einerseits und ex DIIPA roh andererseits war wie folgt (in Gew.-%):

| Komponente | rein EDIIPA ex DIIPA roh | rein EDIIPA ex DIIPA rein |
|---|---|---|
| EDIIPA | 99,840 | 99,891 |
| iPrOH | 0,006 | 0,007 |
| DIIPA | 0,016 | 0,009 |
| DEIPA | 0,001 | 0,003 |
| EIPPA | 0,038 | 0,030 |
| Sonstige | 0,099 | 0,061 |
| iPrOH = iso-Propanol | | |

## Patentansprüche

1. Verfahren zur Herstellung von N-Ethyl-diisopropylamin durch Umsetzung von Acetaldehyd mit Diisopropylamin und Wasserstoff bei erhöhter Temperatur und unter Druck in Gegenwart eines Heterogen-Hydrierkatalysators, wobei es sich bei dem Katalysator um einen geträgerten Übergangsmetallkatalysator handelt, der als katalytisch aktives Metall Pd und/oder Pt enthält, **dadurch gekennzeichnet, dass** das eingesetzte Diisopropylamin zuvor als Nebenprodukt einer Umsetzung von Isopropanol mit Ammoniak zu Monoisopropylamin gewonnen wurde sowie eine Reinheit von 58 bis 94 Gew.-% und Verunreinigungen wie folgt aufweist:

   3 bis 20 Gew.-% Wasser, 3 bis 20 Gew.-% Isopropanol, 0 bis 2 Gew.-% Sonstige.

2. Verfahren nach Anspruch 1, **dadurch gekennzeichnet, dass** das eingesetzte Diisopropylamin eine Reinheit von 62,5 bis 90 Gew.-% und Verunreinigungen wie folgt aufweist: 5 bis 18 Gew.-% Wasser, 5 bis 18 Gew.-% Isopropanol, 0 bis 1,5 Gew.-% Sonstige.

3. Verfahren nach einem der vorhergehenden Ansprüche, **dadurch gekennzeichnet, dass** das Isopropanol zuvor durch Hydrieren von Aceton mit Wasserstoff erhalten wurde.

4. Verfahren nach einem der vorhergehenden Ansprüche, **dadurch gekennzeichnet, dass** das Produkt der Umsetzung von Isopropanol mit Ammoniak durch die nacheinander folgenden Schritte Abtrennung von Ammoniak, Abtrennung von Monoisopropylamin, Abtrennung von höher als Monoisopropylamin und Diisopropylamin siedenden Nebenkomponenten und Abtrennung von ternärem Azeotrop Isopropanol-Diisopropylamin-Wasser destillativ aufgearbeitet wird.

5. Verfahren nach einem der vorhergehenden Ansprüche, **dadurch gekennzeichnet, dass** der geträgerte Übergangsmetallkatalysator Aktivkohle als Träger aufweist.

6. Verfahren nach einem der Ansprüche 1 bis 4, **dadurch gekennzeichnet, dass** der geträgerte Übergangsmetalikatalysator Aluminiumoxid als Träger aufweist.

7. Verfahren nach einem der vorhergehenden Ansprüche, **dadurch gekennzeichnet, dass** der Katalysator einen Gehalt an Pd und/oder Pt im Bereich von 0,1 bis 25 Gew.-% (bezogen auf das Gesamtgewicht des Katalysators, ohne ggf. vorhandenes Wasser) aufweist.

8. Verfahren nach einem der Ansprüche 1 bis 6, **dadurch gekennzeichnet, dass** der Katalysator einen Gehalt an Pd und/oder Pt im Bereich von 0,5 bis 15 Gew.-% (bezogen auf das Gesamtgewicht des Katalysators, ohne ggf. vorhandenes Wasser) aufweist.

9. Verfahren nach einem der vorhergehenden Ansprüche, **dadurch gekennzeichnet, dass** der Katalysator als Suspensionskatalysator eingesetzt wird.

10. Verfahren nach dem vorhergehenden Anspruch, **dadurch gekennzeichnet, dass** die Umsetzung unter Einsatz eines Pd/C-Suspensionskatalysators durchgeführt wird.

11. Verfahren nach einem der vorhergehenden Ansprüche, **dadurch gekennzeichnet, dass** die Umsetzung in Gegenwart von überschüssigem Acetaldehyd durchgeführt wird.

12. Verfahren nach einem der vorhergehenden Ansprüche, **dadurch gekennzeichnet, dass** die Umsetzung bei einer Temperatur im Bereich von 15 bis 180 °C durchgeführt wird.

13. Verfahren nach einem der vorhergehenden Ansprüche, **dadurch gekennzeichnet, dass** die Umsetzung bei einem Druck im Bereich von 1 bis 120 bar durchgeführt wird.

## Claims

1. A process for preparing N-ethyldiisopropylamine by reacting acetaldehyde with diisopropylamine and hydrogen at elevated temperature and under pressure in the presence of a heterogeneous hydrogenation catalyst, the catalyst being a supported transition metal catalyst comprising Pd and/or Pt as catalytically active metal, wherein the diisopropylamine used has been obtained beforehand as a by-product of a reaction of isopropanol with ammonia to give monoisopropylamine and has a purity of 58% to 94% by weight and impurities as follows: 3% to 20% by weight of water, 3% to 20% by weight of isopropanol, 0% to 2% by weight of others.

2. The process according to claim 1, wherein the diisopropylamine used has a purity of 62.5% to 90% by weight and impurities as follows: 5% to 18% by weight of water, 5% to 18% by weight of isopropanol, 0% to 1.5% by weight of others.

3. The process according to either of the preceding claims, wherein the isopropanol has been obtained beforehand by hydrogenating acetone with hydrogen.

4. The process according to any of the preceding claims, wherein the product of the reaction of isopropanol with ammonia is subjected to distillative workup by the successive steps of removing ammonia, removing monoisopropylamine, removing secondary components having higher boiling points than monoisopropylamine and diisopropylamine, and removing ternary isopropanol-diisopropylamine-water azeotrope.

5. The process according to any of the preceding claims, wherein the supported transition metal catalyst includes activated carbon as support.

6. The process according to any of claims 1 to 4, wherein the supported transition metal catalyst includes alumina as support.

7. The process according to any of the preceding claims, wherein the catalyst has a Pd and/or Pt content in the range from 0.1% to 25% by weight (based on the total weight of the catalyst, without any water present).

8. The process according to any of claims 1 to 6, wherein the catalyst has a Pd and/or Pt content in the range from 0.5% to 15% by weight (based on the total weight of the catalyst, without any water present).

9. The process according to any of the preceding claims, wherein the catalyst is used in the form of a suspension catalyst.

10. The process according to the preceding claim, wherein the reaction is conducted using a Pd/C suspension catalyst.

11. The process according to any of the preceding claims, wherein the reaction is conducted in the presence of excess acetaldehyde.

12. The process according to any of the preceding claims, wherein the reaction is conducted at a temperature in the range from 15 to 180°C.

13. The process according to any of the preceding claims, wherein the reaction is conducted at a pressure in the range from 1 to 120 bar.

## Revendications

1. Procédé de fabrication de N-éthyl-diisopropylamine par mise en réaction d'acétaldéhyde avec de la diisopropylamine et de l'hydrogène à température élevée et sous pression en présence d'un catalyseur d'hydrogénation hétérogène, le catalyseur étant un catalyseur de métal de transition supporté qui contient Pd et/ou Pt en tant que métal catalytiquement actif, **caractérisé en ce que** la diisopropylamine utilisée a auparavant été obtenue en tant que produit secondaire d'une réaction d'isopropanol avec de l'ammoniac pour former de la monoisopropylamine, et présente une pureté de 58 à 94 % en poids et les impuretés suivantes :

   3 à 20 % en poids d'eau, 3 à 20 % en poids d'isopropanol, 0 à 2 % en poids d'autres.

2. Procédé selon la revendication 1, **caractérisé en ce que** la diisopropylamine utilisée présente une pureté de 62,5 à 90 % en poids et les impuretés suivantes :

   5 à 18 % en poids d'eau, 5 à 18 % en poids d'isopropanol, 0 à 1,5 % en poids d'autres.

3. Procédé selon l'une quelconque des revendications précédentes, **caractérisé en ce que** l'isopropanol a auparavant été obtenu par hydrogénation d'acétone avec de l'hydrogène.

4. Procédé selon l'une quelconque des revendications précédentes, **caractérisé en ce que** le produit de la réaction d'isopropanol avec de l'ammoniac est traité par distillation par les étapes successives suivantes : séparation de l'ammoniac, séparation de la monoisopropylamine, séparation des composants secondaires ayant un point d'ébullition supérieur à la monoisopropylamine et la diisopropylamine, et séparation de l'azéotrope ternaire isopropanol-diisopropylamine-eau.

5. Procédé selon l'une quelconque des revendications précédentes, **caractérisé en ce que** le catalyseur de métal de transition supporté comprend du charbon actif en tant que support.

6. Procédé selon l'une quelconque des revendications 1 à 4, **caractérisé en ce que** le catalyseur de métal de transition supporté comprend de l'oxyde d'aluminium en tant que support.

7. Procédé selon l'une quelconque des revendications précédentes, **caractérisé en ce que** le catalyseur présente une teneur en Pd et/ou Pt dans la plage allant de 0,1 à 25 % en poids (par rapport au poids total du catalyseur, sans l'eau éventuellement présente).

8. Procédé selon l'une quelconque des revendications 1 à 6, **caractérisé en ce que** le catalyseur présente une teneur en Pd et/ou Pt dans la plage allant de 0,5 à 15 % en poids (par rapport au poids total du catalyseur, sans l'eau éventuellement présente).

9. Procédé selon l'une quelconque des revendications précédentes, **caractérisé en ce que** le catalyseur est utilisé sous la forme d'un catalyseur en suspension.

10. Procédé selon la revendication précédente, **caractérisé en ce que** la réaction est réalisée en utilisant un catalyseur Pd/C en suspension.

11. Procédé selon l'une quelconque des revendications précédentes, **caractérisé en ce que** la réaction est réalisée en présence d'acétaldéhyde en excès.

12. Procédé selon l'une quelconque des revendications précédentes, **caractérisé en ce que** la réaction est réalisée à une température dans la plage allant de 15 à 180 °C.

13. Procédé selon l'une quelconque des revendications précédentes, **caractérisé en ce que** la réaction est réalisée à une pression dans la plage allant de 1 à 120 bar.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9807430 A
- US 2692285 A
- JP 10081650 A
- JP 2851274 B
- JP 02180854 A
- JP 2740828 B
- EP 1020424 A1
- WO 2007137990 A1
- WO 2013075974 A
- EP 696572 A
- EP 563327 A
- WO 9210290 A1


### In der Beschreibung aufgeführte Nicht-Patentliteratur


- *In Chem. Ber.,* 1958, vol. 91, 380-392
- *J. Org. Chem.,* 1951, vol. 16, 1911-1920